# EUROPEAN PATENT APPLICATION

(11) **EP 4 756 007 A1**
(43) Date of publication of application: **10.06.2026**
(21) Application number: 24851763.3
(22) Date of filing: 02.08.2024
(51) Int. Cl.: C12M 1/02, C12M 1/26, C12M 3/00, C12N 1/00

(54) **CELL CULTURING DEVICE AND CELL CULTURING METHOD**

(30) Priority: 04.08.2023 JP 2023127460
(71) Applicant: SHIMADZU CORPORATION, Kyoto-shi, Kyoto 604-8511 (JP); National Institute of Advanced Industrial Science and Technology, Chiyoda-ku Tokyo 100-8921 (JP)
(72) Inventor: TAKAI, Ryogo, Kyoto-shi, Kyoto 604-8511 (JP); NODA, Naohiro, Tsukuba-shi, Ibaraki 305-8561 (JP)
(74) Representative: Müller-Boré & Partner Patentanwälte PartG mbB
(86) International application number: PCT/JP2024/027666
(87) International publication number: WO 2025/033338

(57) **Abstract**

A generation portion (5) generates a dispersion fluid (DI) in which droplets (DR) in which a culture fluid containing cells is sealed are dispersed in a specific solution (S), the specific solution being larger in specific gravity than the culture fluid. A substrate (11) includes a main surface (111), and holds the dispersion fluid (DI) on the main surface (111). A mesh member (2) is arranged away from the main surface (111) of the substrate (11). In the mesh member (2), a plurality of through holes (23) are formed where the droplets (DR) are to be trapped while the mesh member (2) is located above the substrate (11) in a vertical direction, the droplets (DR) having been introduced to the substrate (11) and having risen.

## Description

### TECHNICAL FIELD

The present disclosure relates to a cell culture apparatus and a cell culture method, and more particularly to a technique to culture cells in droplets.

### BACKGROUND ART

A technique to culture cells while water droplets (droplets) in which a culture fluid containing cells is sealed are dispersed in an oil phase has recently attracted attention as one of methods of culturing cells. In the technique, the droplets are each used as an independent culture instrument separated by the oil phase.

NPL 1 and NPL 2 disclose a technique to separate a plurality of droplets in mineral oil smaller in specific gravity than a culture fluid. NPL 1 discloses a technique to introduce mineral oil containing droplets to a microwell chip and to sink droplets in holes in the microwell chip one by one. NPL 2 discloses a technique to sink droplets in mineral oil one by one in a space between posts (pillars) of a microcage array chip. By thus individually capturing (trapping) droplets, desired droplets can be collected, which allows further culture or analysis.

### CITATION LIST

### NON PATENT LITERATURE

NPL 1: Eujin Um et al., "Mesh-integrated microdroplet array for simultaneous merging and storage of single-cell droplets," Lab on a Chip, 2012, 12, pp. 1594-1597.
NPL 2: Jin-Gang Xu et al., "Forming a Large-Scale Droplet Array in a Microcage Array Chip for High-Throughput Screening," Analytical Chemistry, 2019, 91, 16, pp. 10757-10763.

### SUMMARY OF INVENTION

### TECHNICAL PROBLEM

In cell culture using droplets, fluorinated oil is used more desirably than mineral oil in that oxygen is more readily supplied into droplets.

Fluorinated oil, however, is larger in specific gravity than a culture fluid. Therefore, the droplets cannot be separated by sinking as in NPL 1 and NPL 2.

Under such circumstances as above, a technique to enable individual trapping of droplets that float in liquid larger in specific gravity than the culture fluid, such as fluorinated oil, has been demanded.

### SOLUTION TO PROBLEM

A cell culture apparatus according to one aspect of the present disclosure includes a generation portion, a substrate, and a mesh member. The generation portion generates a dispersion fluid in which droplets in which a culture fluid containing cells is sealed are dispersed in a specific solution, the specific solution being larger in specific gravity than the culture fluid. The substrate includes a main surface, and holds the dispersion fluid on the main surface. The mesh member is arranged away from the main surface of the substrate. In the mesh member, a plurality of through holes are formed where the droplets are to be trapped while the mesh member is located above the substrate in a vertical direction, the droplets having been introduced to the substrate and having risen.

A cell culture method according to another aspect of the present disclosure includes generating a dispersion fluid in which droplets in which a culture fluid containing cells is sealed are dispersed in a specific solution, the specific solution being larger in specific gravity than the culture fluid, holding, at a substrate including a main surface, the dispersion fluid on the main surface, and arranging a mesh member away from the main surface of the substrate. In the mesh member, a plurality of through holes are formed where the droplets are to be trapped while the mesh member is located above the substrate in a vertical direction, the droplets having been introduced to the substrate and having risen.

### ADVANTAGEOUS EFFECTS OF INVENTION

According to the culture apparatus of the present disclosure, droplets that float in a specific solution larger in specific gravity than a culture fluid can individually be trapped.

### BRIEF DESCRIPTION OF DRAWINGS

Fig. 1 is a schematic configuration diagram of a culture unit according to an embodiment.
Fig. 2 is a cross-sectional view of a culture portion according to the embodiment.
Fig. 3 is a diagram showing exemplary overview of an observation image of droplets in the culture portion.
Fig. 4 is a flowchart showing an exemplary culture method according to the embodiment.
Fig. 5 is a diagram for illustrating trapping droplets.
Fig. 6 is a perspective view showing a culture system according to another embodiment.
Fig. 7 is a diagram of a structure around the culture portion and a linear guide viewed from obliquely above.
Fig. 8 is a cross-sectional view showing the structure of the culture portion.
Fig. 9 is a cross-sectional view showing the structure around the culture portion.

### DESCRIPTION OF EMBODIMENTS

An embodiment of the present disclosure will be described in detail below with reference to the drawings. The same or corresponding elements in the drawings have the same reference characters allotted and description thereof will not be repeated.

### [Configuration of Culture Unit and Culture Apparatus]

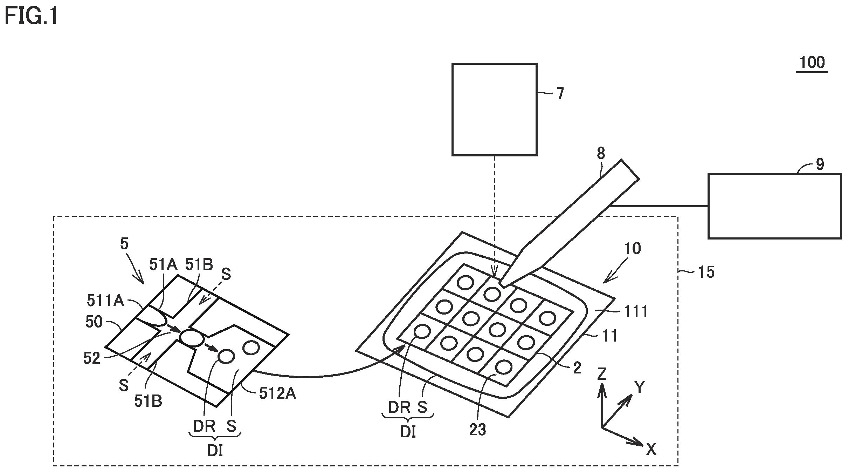

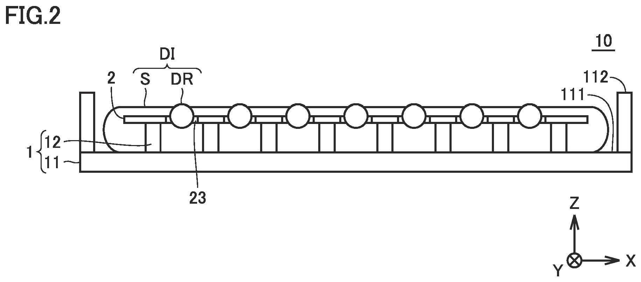

Fig. 1 is a schematic configuration diagram of a culture unit 100 according to the present embodiment. Culture unit 100 includes a microscope 7, a capillary 8, a control device 9, and a culture apparatus 15. Culture apparatus 15 corresponds to one example of the "cell culture apparatus." Culture apparatus 15 includes a generation portion 5 and a culture portion 10. Fig. 2 is a cross-sectional view of culture portion 10 according to the embodiment. Culture portion 10 includes a substrate 11 and a mesh member 2. Figs. 1 and 2 are diagrams of culture unit 100 and culture portion 10 with droplets DR being trapped in mesh member 2. In Figs. 1 and 2, a direction perpendicular to substrate 11 is defined as a Z axis and a plane in parallel to substrate 11 is defined as an XY plane. In one example, culture portion 10 is used as being set such that the Z axis is substantially in parallel to a direction of gravity (vertical direction).

Generation portion 5 generates a dispersion fluid DI in which droplets DR in which a culture fluid containing cells is sealed are dispersed in a specific solution S, specific solution S being larger in specific gravity than the culture fluid. In one example, generation portion 5 includes a micro flow channel device 50 where micro flow channels 51A and 51B are formed. Micro flow channel 51A and micro flow channel 51B intersect with each other at an intersection position 52. A prescribed amount of culture fluid containing cells is intermittently fed from an end 511A of micro flow channel 51A. Specific solution S is fed from micro flow channel 51B. More specifically, specific solution S is supplied from micro flow channel 51B to micro flow channel 51A in such a manner that the specific solution flows between opposing sides. Droplets DR in which the prescribed amount of culture fluid has been sealed are thus generated in specific solution S. In other words, dispersion fluid DI in which droplets DR are dispersed in specific solution S is generated. Dispersion fluid DI then flows out from an end 512A. End 512A is located opposite to end 511A of micro flow channel 51A with intersection position 52 lying therebetween. Dispersion fluid DI is introduced to culture portion 10, for example, by manual operations by a user. Culture unit 100 may include an apparatus (not shown) that transports dispersion fluid DI from generation portion 5 to culture portion 10, and the apparatus may be controlled by control device 9.

In culture portion 10, dispersion fluid DI is introduced to a main surface 111 of substrate 11 and thereafter held thereon. Main surface 111 is a surface of substrate 11 on a side of a positive direction of the Z axis (upper side in the vertical direction). Specific solution S is larger in specific gravity than the culture fluid. Mesh member 2 is arranged away from main surface 111 of substrate 11. In mesh member 2, a plurality of through holes 23 are formed where droplets DR are to be trapped while mesh member 2 is located above substrate 11 in the vertical direction, the droplets having been introduced to substrate 11 and having risen. More specifically, in mesh member 2, a plurality of through holes 23 of a size that allows individual trapping of droplets DR are formed. According to such a configuration, each of droplets DR that float in specific solution S rises in through holes 23. In other words, droplets DR can individually be trapped in through holes 23. Therefore, cells can be cultured and observed while the cells are isolated for each droplet DR.

In one example, cells are microorganisms. Some of microorganisms in the environment are closely involved with humans, and used not only in daily life but also in industries, engineering, or the like. Most of microorganisms in the environment, however, have not successfully been cultured and not effectively been used as biological resources. Physiological characteristics of many of microorganisms in a prescribed environment are also unclear. Therefore, culture of microorganisms to clarify properties thereof may be applied to development of medicine, industries, or the like. In another example, cells are animal cells. In this case again, culture of animal cells to clarify properties thereof may be applied to development of medicine, industries, or the like. In yet another example, cells are plant cells.

In one example, the culture fluid contains cells and a liquid medium. The culture fluid may contain a marker for visually showing characteristics or an amount of cells or a metabolite generated by cells. The marker is, for example, a substance indicating presence of a prescribed metabolite by color or fluorescence.

Droplets DR are water droplets having a diameter, for example, from approximately 30 to 130 µm, although they are not limited as such. By using a dedicated apparatus, droplets DR in specific solution S can be prepared in batches of several hundred thousand at a time.

In one example, specific solution S is fluorinated oil. Examples of fluorinated oil include FC40, Novec^{™} (the same hereafter) 7500, combinations thereof, and the like. Fluorinated oil is higher than mineral oil in oxygen permeability. In addition, there is a surfactant suitable for making droplets DR in fluorinated oil. The user adds the surfactant to at least one of an aqueous phase (in the culture fluid) and an oil phase (in fluorinated oil). For example, the user uses Span^{®}80, Tween^{®}20, or the like as the surfactant for the aqueous phase. Alternatively, for example, the user uses 008-FluoroSurfactant, Pico-surf^{™} 1, Krytox^{™}, or the like as the surfactant for the oil phase. By using fluorinated oil as specific solution S as above, oxygen necessary for culture of cells can readily be supplied into droplets DR. In addition, with the surfactant, droplets DR can be maintained as independent compartments for a long time and in a stable manner, without droplets DR coalescing. Cells are thus readily cultured and observed for a long period while they are isolated for each droplet DR. Specific solution S is not limited to the examples above, and a liquid having a specific gravity larger than that of the culture fluid may be applicable. For example, ionic liquid or liquid mercury can be employed as liquid larger in specific gravity than the culture fluid. Specific solution S is a liquid that is not miscible with the culture fluid. In one example, an amount of dispersion fluid DI introduced to substrate 11 is an amount necessary and sufficient for trapping droplets DR in through holes 23. Specifically, for example, the amount is such an amount that a height of a fluid level of dispersion fluid DI from substrate 11 is substantially the same as a height of mesh member 2. For example, a micropillar of Yodaka Co., Ltd. can be employed as a micropillar device 1. Micropillar device 1 may be made, for example, by casting using polydimethylsiloxane.

In one example, culture portion 10 further includes a protruding portion 12 that is arranged on substrate 11 and protrudes upward from substrate 11. Mesh member 2 is arranged above protruding portion 12. According to such a configuration, mesh member 2 is readily arranged above substrate 11 away therefrom. More specifically, it is easy to keep a distance between mesh member 2 and substrate 11 at a certain value or larger. In the example in Fig. 2, an upper end of protruding portion 12 and mesh member 2 are in contact with each other and protruding portion 12 directly supports mesh member 2. When dispersion fluid DI is located between substrate 11 and mesh member 2, however, dispersion fluid DI may be introduced in between mesh member 2 and protruding portion 12 and mesh member 2 may be distant from protruding portion 12 (floating over dispersion fluid DI). Substrate 11 and protruding portion 12 are preferably integrally formed. According to such a configuration, mesh member 2 is readily held above substrate 11 away therefrom in a stable manner, and handling of culture portion 10 as a whole is also facilitated. In the example in Fig. 2, micropillar device 1 including substrate 11 and protruding portion 12 is used. The configuration for arranging mesh member 2 above substrate 11 away therefrom in culture portion 10, however, is not limited to the example above. Mesh member 2 and protruding portion 12 may integrally be formed, or all of substrate 11, protruding portion 12, and mesh member 2 may integrally be formed. Culture portion 10 may include, instead of protruding portion 12, a feature that laterally clamps mesh member 2 and holds the mesh member at a prescribed height from substrate 11.

In one example, substrate 11 is a container provided with a wall 112 at a peripheral portion thereof, the wall standing upward in the vertical direction. By providing wall 112 at the peripheral portion of substrate 11, possibility of spillage of dispersion fluid DI over the peripheral portion becomes lower than in an example where wall 112 is not provided.

Mesh member 2 is typically a plate-shaped member where a plurality of through holes 23 are formed, the through holes each having a size that allows individual trapping of droplets DR. An exemplary mesh member 2 is a net-like member meshed in a grid pattern. In this case, such grids correspond to through holes 23. In one example, mesh member 2 is selected such that a ratio of a diameter of droplet DR to the grid opening is not lower than 80% and not higher than 120%, preferably not lower than 90% and not higher than 110%. Another exemplary mesh member 2 is a member provided with circular through holes 23. Preferably, through holes 23 in mesh member 2 are regularly arranged at substrate 11. Identification and continual observation of each of droplets DR trapped by mesh member 2 are thus facilitated.

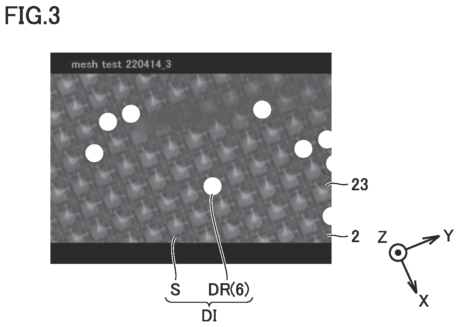

Microscope 7 is an apparatus for observation of droplets DR in culture portion 10. Microscope 7 is, for example, an optical microscope. Culture portion 10 is placed at rest on a not-shown stage, and droplets DR in culture portion 10 are observed through a not-shown lens. Fig. 3 is a diagram showing an exemplary overview of an observation image of droplets DR in culture portion 10. In the observation image in Fig. 3, portions corresponding to droplets DR are labeled with white circles 600. As shown in Fig. 3, the user can observe from above, droplets DR trapped in through holes 23 in mesh member 2, as being enlarged.

While observation with microscope 7 is not made, culture portion 10 may be accommodated in a not-shown incubator. In one example, culture portion 10 may be accommodated for a prescribed time period not shorter than several hours and not longer than several ten hours in an incubator set to a prescribed temperature not lower than 25°C and not higher than 40°C and then observation with microscope 7 may be made. In the example, culture portion 10 is preferably accommodated after it is subjected to a drying prevention process. For example, a process to cover an upper surface of culture portion 10 with a cover or the like can be performed as the drying prevention process.

In one example, capillary 8 is moved under the control of control device 9 to individually collect (pick) desired droplet DR. According to such a configuration, accurate and fine control of the position of capillary 8 is easier than in an example where the user manually moves capillary 8. Control device 9 may be configured to move the tip end of capillary 8 to a position of droplet DR located at a prescribed position in the field of view of microscope 7, in coordination with microscope 7. Capillary 8 corresponds to one example of the "collection apparatus." A capillary having an inner diameter at a tip end thereof as large as the diameter of droplet DR can be employed, although capillary 8 is not limited as such. Capillary 8 is capable of suctioning even a droplet DR having a diameter approximately a prescribed ratio larger than through hole 23 in mesh member 2 (for example, the diameter 110% to 120% the size of through hole 23). Capillary 8 may be configured to suction droplets DR based on a capillary phenomenon or to suction droplets DR at a prescribed amount of suction by using a suctioning portion (not shown) that supplies suction force.

The user can transfer cells contained in droplets DR collected by capillary 8 to another container and perform further culture, observation, and/or analysis.

### [Comparison with Conventional Method of Culturing Cells Using Droplets]

A technique to culture cells by using small droplets (water-in-oil droplets) dispersed in the oil phase has recently attracted attention. With the technique, many types of microorganisms can be cultured in a small space. In this connection, NPLs 1 and 2 disclose a technique to separate droplets in mineral oil smaller in specific gravity than the culture fluid.

NPL 1 discloses a technique to use a microwell chip and a mesh for isolating and aligning droplets generated in mineral oil. Specifically, the mesh is arranged on a microwell plate where a spacer is arranged at an upper surface. The mesh helps in filling microwells with microdroplets.

NPL 2 discloses a technique to use a microcage array chip for isolating and aligning microdroplets generated in mineral oil. Specifically, by spreading mineral oil containing droplets over the microcage array chip having a pillar structure, droplets sink among pillars.

In culture of cells in droplets, on the other hand, use of fluorinated oil rather than mineral oil is preferable because oxygen is more readily supplied into droplets and a surfactant that stabilizes droplets for a long period is available. Unlike mineral oil, however, fluorinated oil is larger in specific gravity than the culture fluid. Therefore, the techniques described in NPLs 1 and 2 are techniques for trapping droplets that sink in mineral oil smaller in specific gravity than the culture fluid, and they are inapplicable to droplets floating in fluorinated oil. From the foregoing, a technique to isolate droplets floating in liquid larger in specific gravity than the culture fluid, such as fluorinated oil, has been demanded.

There are problems as described below for isolating droplets floating in fluorinated oil. Initially, positions of droplets floating in fluorinated oil are unstable and it is difficult to isolate an intended droplet. In addition, droplets floating over fluorinated oil coalesce and it is difficult to manipulate a single droplet. Furthermore, droplets should be in such a state that they can be suctioned by the capillary.

Then, in culture portion 10 according to the present embodiment, mesh member 2 is set over substrate 11 and dispersion fluid DI in which droplets DR are dispersed in specific solution S such as fluorinated oil is introduced to substrate 11. Droplets DR can thus rise in through holes 23 in mesh member 2 and individually be trapped therein. Droplets DR in specific solution S can thus be dispersed and fixed. Furthermore, capillary 8 can be brought into contact with droplets DR from above through hole 23 for suction.

### [Flowchart of Cell Culture Method]

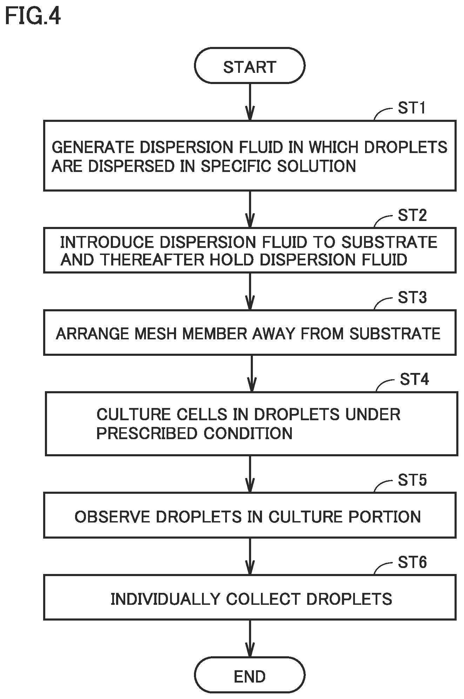

Fig. 4 is a flowchart showing an exemplary cell culture method according to the embodiment.

In step (which will be denoted as "ST" below) 1, the user generates dispersion fluid DI in which droplets DR in which the culture fluid containing cells is sealed are dispersed in specific solution S. Specific solution S is larger in specific gravity than the culture fluid.

In ST2, the user introduces, at substrate 11 including main surface 111, dispersion fluid DI to main surface 111 and thereafter has the dispersion fluid held thereon.

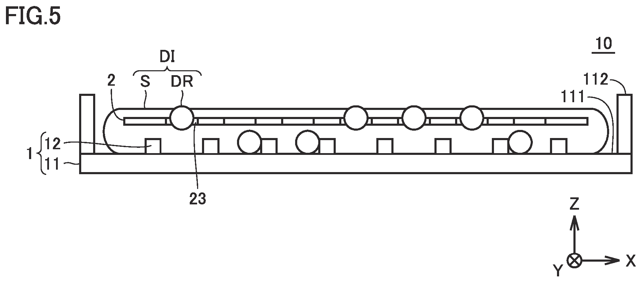

In ST3, the user arranges mesh member 2 away from main surface 111 of substrate 11. In mesh member 2, a plurality of through holes 23 where droplets DR are to be trapped while mesh member 2 is located above substrate 11 in the vertical direction are formed, the droplets having been introduced to substrate 11 and having risen. In one example, the user arranges mesh member 2 above protruding portion 12 that is arranged on substrate 11 and protrudes upward from the substrate (Fig. 5). After the user arranges mesh member 2, in order to promote dispersion and/or rise of droplets DR in specific solution S and to facilitate individual trap in through holes 23, specific solution S or dispersion fluid DI may be added to culture portion 10 or moderate vibration may be applied to culture portion 10. Preferably, the fluid level of dispersion fluid DI introduced in culture portion 10 is adjusted to be located between a lower surface of mesh member 2 and a position higher by a radius of droplet DR from an upper surface of mesh member 2. In other words, dispersion fluid DI in an amount between a lower limit value and an upper limit value which will be described below is preferably introduced in culture portion 10. The lower limit value is a minimum amount at which dispersion fluid DI and mesh member 2 come in contact with each other and the upper limit value is an amount at which the fluid level of dispersion fluid DI is located at a position higher by the radius of droplet DR from the upper surface of mesh member 2.

In ST4, the user cultures cells in droplets DR under a prescribed condition. In one example, the user places culture portion 10 at rest for a prescribed time period in an incubator set to a prescribed temperature.

In ST5, the user observes droplets DR in culture portion 10. In one example, the user observes with microscope 7, droplets DR trapped in through holes 23 in ST3. In one example, the user selects droplets DR to be collected in ST6, based on a result of observation.

In ST6, the user individually collects droplets DR. In one example, the user brings capillary 8 into contact with any droplet DR selected in observation in ST5 and suctions the droplet. The user then transfers suctioned droplet DR to a prescribed container and performs further culture and/or analysis.

In the process in Fig. 4, the order of ST2 and ST3 may be reverse.
Specifically, mesh member 2 may be arranged above substrate 11 away therefrom and thereafter dispersion fluid DI may be introduced and held between mesh member 2 and substrate 11. In an example where culture portion 10 structured such that micropillar device 1 and mesh member 2 are integrated as above is used, mesh member 2 has been arranged above substrate 11 at a time point of ST2 and hence operation in ST3 is omitted. After some of droplets DR are collected from culture portion 10, culture and/or observation of droplets DR that remain in culture portion 10 may be continued. At least a part of the process in Fig. 4 may be automated by an apparatus for experiments, and the apparatus may be controlled by control device 9.

According to the process in Fig. 4, positions of droplets DR can be fixed, with droplets DR in specific solution S being dispersed. In addition, while fixed droplets DR are observed with the microscope, any droplets DR can be suctioned by capillary 8. Specifically, a difference in state in droplets DR originating from the type of sealed cells can be observed and droplets DR containing a preferred type of cells can be collected. For example, the user can observe turbidity, a color, or the like of droplets DR and isolate cells high in growth rate, cells high in yield of a prescribed metabolite, or the like. The cell culture method according to the present embodiment can thus be concluded as an efficient screening method for readily selecting and culturing a desired type of cells from among a large number of types of cells cultured in a space saving manner.

### [Configuration of Culture System]

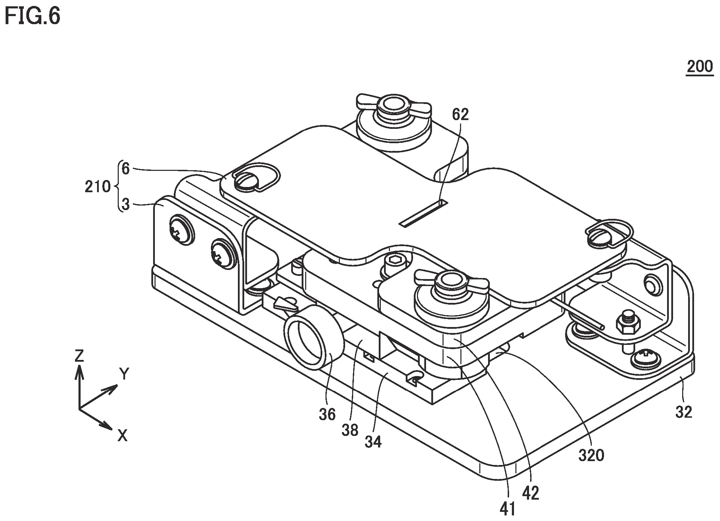

A culture system 200 configured to suppress decrease in specific solution S due to drying during culture and observation of cells will then be described with reference to Figs. 6 to 9. Culture system 200 is used as a culture unit together with microscope 7, capillary 8, control device 9, and generation portion 5.

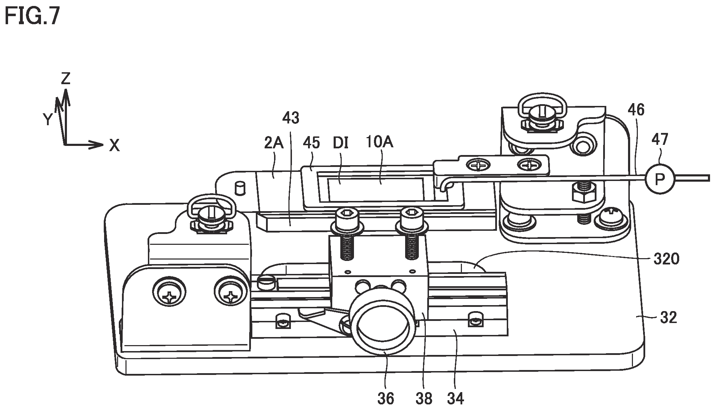

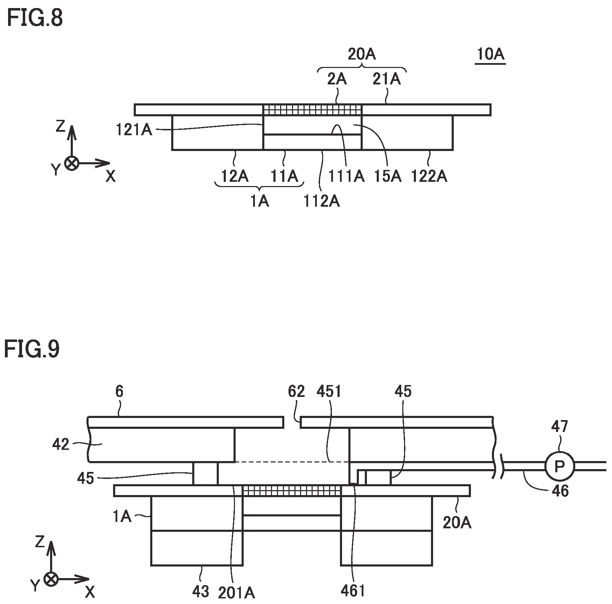

Fig. 6 is a perspective view showing culture system 200 according to another embodiment. Fig. 7 is a diagram of a structure around a culture portion 10A and a linear guide 34 viewed from obliquely above, with a lid 6, a first connection member 41, and a second connection member 42 having been removed from culture system 200 in Fig. 6. Fig. 8 is a cross-sectional view showing the structure of culture portion 10A. Fig. 9 is a cross-sectional view showing the structure around culture portion 10A.

Culture system 200 includes culture portion 10A and a main body 210 where culture portion 10A is accommodated. Main body 210 includes lid 6 and a base unit 3.

Base unit 3 includes a base 32, linear guide 34, a handle 36, a carriage 38, first connection member 41, second connection member 42, a third connection member 43, a spacer 45, an introduction pipe 46, and a pump 47.

In Fig. 6 or following figures, a direction of extension of linear guide 34 is defined as an X axis, a direction perpendicular to base 32 is defined as the Z axis, and a plane in parallel to base 32 is defined as the XY plane. In one example, culture system 200 is used in such a state that the Z axis is substantially in parallel to the vertical direction. For example, culture system 200 is placed at rest in an incubator or the like for culture or at an observation stage of microscope 7 for observation, with the Z axis substantially being in parallel to the vertical direction.

Culture portion 10A includes a mesh device 20A and a substrate device 1A.

Substrate device 1A includes a substrate 11A and a substrate holding portion 12A.

Substrate 11A includes a main surface 111A and holds dispersion fluid DI on main surface 111A. In one example, substrate 11A is a plate-shaped member formed of transparent glass.

Substrate holding portion 12A holds substrate 11A. In one example, substrate holding portion 12A is a plate-shaped member where a through hole 121A is formed, and substrate 11A is fitted in through hole 121A.

In one example, a thickness (a length in a Z-axis direction) of substrate holding portion 12A is formed to be larger than a thickness (the length in the Z-axis direction) of substrate 11A. According to such a configuration, when the substrate is fitted such that positions in the Z-axis direction of a lower surface 122A of substrate holding portion 12A and a lower surface 112A of substrate 11A are aligned (flush with each other), a recess 15A having a bottom surface defined by main surface 111A of substrate 11A and surrounded by substrate holding portion 12A is formed in substrate device 1A. Dispersion fluid DI is introduced in that recess 15A.

Mesh device 20A includes a mesh member 2A and a mesh holding portion 21A. Mesh device 20A is placed on substrate device 1A such that mesh holding portion 21A is located above substrate 11A. Mesh member 2A is thus arranged away from main surface 111A of substrate 11A. Substrate holding portion 12A of substrate device 1A also corresponds to one example of the "protruding portion."

In mesh member 2A, a plurality of through holes are formed where droplets DR are to be trapped while mesh member 2A is located above substrate 11A in the vertical direction, the droplets having been introduced to substrate 11A and having risen.

In one example, mesh device 20A is a plate-shaped member in which mesh member 2A is formed at a position corresponding to substrate 11A when mesh device 20A is arranged above substrate device 1A.

According to such a configuration, each of droplets DR floating in specific solution S rises in the through hole in mesh member 2A. In other words, droplets DR can individually be trapped in the through holes. Therefore, cells can be cultured and observed while the cells are isolated for each droplet DR.

Referring to Fig. 9, spacer 45 is set at an upper surface of mesh holding portion 21A so as to surround an upper surface of mesh member 2A. In one example, spacer 45 is a plate-shaped member including a through hole a size larger than mesh member 2A, and arranged on mesh device 20A such that the through hole is located above mesh member 2A. With spacer 45, even when dispersion fluid DI flows out to the upper surface of mesh holding portion 21A at the time when mesh device 20A is laid over substrate device 1A, the flow can be stopped by spacer 45. A space where dispersion fluid DI is introduced, that is located below an upper end 451 of spacer 45, is also referred to as a "pool".

Introduction pipe 46 is an introduction flow channel for introduction of specific solution S, the introduction flow channel being composed, for example, of silicon and being fluidly connected to main body 210. By way of example, introduction pipe 46 has a tip end 461 arranged in contact with the vicinity of an upper surface 201A of mesh device 20A at an inner side of spacer 45. In this case, a plurality of cuts are provided at tip end 461 in a circumferential direction. Alternatively, tip end 461 is cut to be tapered. By thus configuring tip end 461, even when specific solution S is introduced at a low flow rate, liquid droplets of specific solution S can be prevented from remaining at tip end 461 and specific solution S can quickly be diffused in the pool. In another example, tip end 461 is arranged away from upper surface 201A at such a prescribed distance that liquid droplets are not away from upper surface 201A when liquid droplets are produced at tip end 461. In this case, a plurality of cuts at tip end 461 in the circumferential direction or tapered cut of tip end 461 are/is not necessarily required. According to such a configuration again, even when specific solution S is introduced at a low flow rate, liquid droplets of specific solution S can be prevented from remaining at tip end 461 and specific solution S can quickly be diffused in the pool.

Pump 47 is connected to an end opposite to tip end 461 of introduction pipe 46 and delivers specific solution S to introduction pipe 46. Pump 47 is, for example, a syringe pump.

With a mechanism to dispense specific solution S to main body 210, such as introduction pipe 46 and pump 47, cells can be cultured and observed for a long period with new supply of dispersion fluid DI in an amount compensating for shortage caused by drying.

In one example, the position of tip end 461 of introduction pipe 46 is fixed with respect to upper surface 201A of mesh device 20A. Thus, as will be described later, also when culture portion 10A including mesh device 20A is translated, the position of tip end 461 with respect to the position of upper surface 201A is held.

Second connection member 42 is joined to an upper portion of spacer 45. In one example, second connection member 42 is a plate-shaped member including a through hole a size larger than mesh member 2A and arranged on spacer 45 such that the through hole is located above mesh member 2A. Second connection member 42 has an upper surface arranged in contact with a lower surface of lid 6.

Spacer 45 and second connection member 42 seal a gap between culture portion 10A and lid 6, and evaporation of the specific solution through the gap can be suppressed.

In lid 6, an opening 62 is formed. In one example, lid 6 is a plate-shaped member including opening 62 and having a size enough to constantly cover an upper portion of culture portion 10A even when culture portion 10A is translated.

Opening 62 is a slit smaller in width than mesh member 2A. The "width" herein means a length in an X direction. Opening 62 is configured to have a width that allows insertion of capillary 8 therethrough for collection or observation with microscope 7. By thus providing lid 6 provided with opening 62 smaller in width than mesh member 2A, suppression of drying of dispersion fluid DI and suitable observation and collection of cells can both be achieved.

Preferably, by configuring opening 62 to have a minimum width (for example, several millimeters) that allows insertion of capillary 8 therethrough for collection or observation with microscope 7, drying of dispersion fluid DI can be suppressed to a maximum extent while cells are suitably observed and collected.

Relative positional relation between mesh member 2A and opening 62 of lid 6 is changed in a horizontal direction perpendicular to the vertical direction by linear guide 34, handle 36, and carriage 38. Linear guide 34, handle 36, and carriage 38 correspond to one example of the "translation mechanism." "Translation" herein refers to parallel movement in an X-axis direction along the linear guide.

Linear guide 34 is fixed on base 32. Carriage 38 translates over the linear guide. With handle 36, a position of carriage 38 over linear guide 34 is controlled.

Carriage 38 and culture portion 10A are connected to each other by connection members 41 to 43.

In one example, connection members 41 to 43 are configured as below. First connection member 41 is a plate-shaped member provided with a through hole where third connection member 43 is to be buried. First connection member 41 is fixed to an upper surface of carriage 38. After dispersion fluid DI is introduced to culture portion 10A, second connection member 42 is fixed to a part of an upper surface of first connection member 41. Third connection member 43 is a plate-shaped member to be fitted into first connection member 41. Substrate device 1A of culture portion 10A is fixed to an upper surface of third connection member 43. According to such a configuration, as carriage 38 over linear guide 34 is translated, culture portion 10A also translates.

As above, linear guide 34, handle 36, and carriage 38 can be used to change the position of mesh member 2A with respect to opening 62 of lid 6 in the horizontal direction. Therefore, at mesh member 2A, cells located at a desired position can be observed and collected.

Preferably, in culture system 200, substrate 11A is formed of a material having light transmittance not lower than a prescribed value, and a member vertically below substrate 11A is also configured to allow passage of light therethrough. For example, substrate 11A is formed of a transparent glass material, third connection member 43 is also provided with a through hole 430 at a position corresponding to substrate 11A (a position having the same XY coordinates), and base 32 is also provided with a through hole 320 at a position corresponding to substrate 11A. A through hole is formed also at a position in first connection member 41 corresponding to substrate 11A, or a recess for passage of light is formed (not-shown) at a position corresponding to substrate 11A. In mesh member 2A of mesh device 20A, a plurality of through holes are formed and configured to allow passage of light therethrough. According to such a configuration, light that enters a bottom surface of main body 210 can be emitted to culture portion 10A. Culture portion 10A is thus readily observed from above opening 62 with the microscope.

In culture system 200, the user arranges dispersion fluid DI at substrate device 1A, thereafter places mesh member 2A, and closes lid 6 to perform culture and observation.

In a more specific example, the user arranges dispersion fluid DI at substrate device 1A connected to carriage 38 and thereafter fixes mesh member 2A, spacer 45, and second connection member 42 to substrate device 1A. Furthermore, lid 6 is fixed to base 32. According to such a configuration, with drying of dispersion fluid DI being minimized, cells can be cultured or cells at each position in mesh member 2A can be observed or collected.

The configuration of each component of culture system 200 may be modified as appropriate within the scope where effects above are achieved.

For example, an outer shape of at least one of lid 6 and connection members 41 to 43 may be quadrangular. At least two of substrate device 1A, third connection member 43, first connection member 41, and carriage 38 may integrally be formed. At least two of mesh member 2A, spacer 45, and second connection member 42 may integrally be formed.

Movement of carriage 38 with respect to linear guide 34 may be realized by manually turning handle 36 or by driving an actuator. Carriage 38 over linear guide 34 may be translated by pushing carriage 38 in the X-axis direction, without providing handle 36.

A drain pipe (not-shown) for draining oil from the pool may additionally be provided.

### [Aspects]

Illustrative embodiments described above are understood by a person skilled in the art as specific examples of aspects below.

(Clause 1) A cell culture apparatus according to one aspect includes a generation portion, a substrate, and a mesh member. The generation portion generates a dispersion fluid in which droplets in which a culture fluid containing cells is sealed are dispersed in a specific solution, the specific solution being larger in specific gravity than the culture fluid. The substrate includes a main surface, and holds the dispersion fluid on the main surface. The mesh member is arranged away from the main surface of the substrate. In the mesh member, a plurality of through holes are formed where the droplets are to be trapped while the mesh member is located above the substrate in a vertical direction, the droplets having been introduced to the substrate and having risen.

According to the cell culture apparatus described in Clause 1, each of droplets floating in the specific solution larger in specific gravity than the culture fluid rises in the through hole. The droplets can thus individually be trapped in the through holes in the mesh member.

(Clause 2) The cell culture apparatus described in Clause 1 further includes a protruding portion arranged on the substrate, the protruding portion protruding upward from the substrate. The mesh member is arranged above the protruding portion.

According to the cell culture apparatus described in Clause 2, the mesh member is readily arranged above the substrate away therefrom. More specifically, a distance between the mesh member and the substrate is readily kept in a stable manner.

(Clause 3) In the cell culture apparatus described in Clause 1 or 2, the specific solution includes fluorinated oil.

According to the cell culture apparatus described in Clause 3, cells are readily cultured and observed for a long period while the cells are isolated for each droplet.

(Clause 4) In the cell culture apparatus described in Clause 2, the substrate and the protruding portion are integrally formed.

According to the cell culture apparatus described in Clause 4, the mesh member is readily held above the substrate away therefrom in a stable manner, and handling of the cell culture apparatus as a whole is also facilitated.

(Clause 5) In the cell culture apparatus described in any one of Clauses 1 to 4, the cells include microorganisms or animal cells.

According to the cell culture apparatus described in Clause 5, culture of microorganisms to clarify properties thereof may be applied to development of medicine, industries, or the like. Alternatively, culture of animal cells to clarify properties thereof may be applied to development of medicine, industries, or the like.

(Clause 6) The cell culture apparatus described in any one of Clauses 1 to 5 further includes a main body where the dispersion fluid is accommodated, the main body holding the substrate and the mesh member and an introduction flow channel for introduction of the specific solution, the introduction flow channel being fluidly connected to the main body.

According to the cell culture apparatus described in Clause 6, with a mechanism to dispense the specific solution to the main body, cells can be cultured and observed for a long period with new supply of the dispersion fluid in an amount compensating for shortage thereof caused by drying.

(Clause 7) The cell culture apparatus described in any one of Clauses 1 to 6 further includes a lid arranged above the mesh member in the vertical direction, and in the lid, an opening smaller in width than the mesh member is formed.

According to the cell culture apparatus described in Clause 7, by providing the lid provided with the opening smaller in width than the mesh member, suppression of drying of the dispersion fluid and suitable observation and collection of cells can both be achieved.

(Clause 8) The cell culture apparatus described in Clause 7 further includes a translation mechanism that changes a position of the mesh member with respect to the opening of the lid in a horizontal direction.

According to the cell culture apparatus described in Clause 8, by changing relative positional relation between the mesh member and the opening of the lid, cells located at a desired position can be observed and collected.

(Clause 9) A culture unit includes the cell culture apparatus described in any one of Clauses 1 to 8, a microscope for observation of the droplets in the cell culture apparatus, and a collection apparatus that individually collects the droplets.

According to the culture unit described in Clause 9, the user can observe from above, droplets trapped in the through holes in the mesh member, as being enlarged. The user can then individually collect desired droplets with a capillary. Furthermore, the user can transfer cells contained in droplets collected by the capillary to another container and perform further culture, observation, and/or analysis.

(Clause 10) A cell culture method according to another aspect includes generating a dispersion fluid in which droplets in which a culture fluid containing cells is sealed are dispersed in a specific solution, the specific solution being larger in specific gravity than the culture fluid, holding, at a substrate including a main surface, the dispersion fluid on the main surface, and arranging a mesh member away from the main surface of the substrate. In the mesh member, a plurality of through holes are formed where the droplets are to be trapped while the mesh member is located above the substrate in a vertical direction, the droplets having been introduced to the substrate and having risen.

According to the cell culture method described in Clause 10, each of droplets floating in the specific solution larger in specific gravity than the culture fluid rises in the through hole. The droplets can thus individually be trapped in the through holes in the mesh member.

(Clause 11) In the cell culture method described in Clause 10, the arranging the mesh member includes arranging the mesh member above a protruding portion that is arranged on the substrate and protrudes upward from the substrate.

According to the cell culture method described in Clause 11, the mesh member is readily arranged above the substrate away therefrom. More specifically, a distance between the mesh member and the substrate is readily kept in a stable manner.

(Clause 12) The cell culture method described in Clause 10 or 11 further includes observing the droplets trapped in the mesh member and individually collecting the droplets.

According to the cell culture method described in Clause 12, the user can observe from above with a microscope or the like, droplets trapped in the through holes in the mesh member. The user can then individually collect desired droplets with a capillary. Furthermore, the user can transfer cells contained in droplets collected by the capillary or the like to another container and perform further culture, observation, and/or analysis.

It should be understood that the embodiment disclosed herein is illustrative and non-restrictive in every respect. The scope of the present disclosure is defined by the terms of the claims rather than the description of the embodiment above and is intended to include any modifications within the scope and meaning equivalent to the terms of the claims.

### REFERENCE SIGNS LIST

1 micropillar device; 1A substrate device; 2, 2A mesh member; 3 base unit; 5 generation portion; 6 lid; 7 microscope; 8 capillary; 9 control device; 10, 10A culture portion; 11, 11A substrate; 12 protruding portion; 12A substrate holding portion; 15 culture apparatus; 15A recess; 20A mesh device; 21A mesh holding portion; 23, 121A, 320, 430 through hole; 32 base; 34 linear guide; 36 handle; 38 carriage; 41 first connection member; 42 second connection member; 43 third connection member; 45 spacer; 46 introduction pipe; 47 pump; 50 micro flow channel device; 51A, 51B micro flow channel; 52 intersection position; 62 opening; 100, 100A culture unit; 111, 111A main surface; 112 wall; 112A, 122A lower surface; 200 culture system; 201A upper surface; 210 main body; 451 upper end; 461 tip end; 511A, 512A end; DI dispersion fluid; DR droplet; S specific solution.

## Claims

1. A cell culture apparatus comprising:
a generation portion that generates a dispersion fluid in which droplets in which a culture fluid containing cells is sealed are dispersed in a specific solution, the specific solution being larger in specific gravity than the culture fluid;
a substrate including a main surface, the substrate holding the dispersion fluid on the main surface; and
a mesh member arranged away from the main surface of the substrate, wherein
in the mesh member, a plurality of through holes are formed where the droplets are to be trapped while the mesh member is located above the substrate in a vertical direction, the droplets having been introduced to the substrate and having risen.

2. The cell culture apparatus according to claim 1, further comprising a protruding portion arranged on the substrate, the protruding portion protruding upward from the substrate, wherein
the mesh member is arranged above the protruding portion.

3. The cell culture apparatus according to claim 1, wherein
the specific solution includes fluorinated oil.

4. The cell culture apparatus according to claim 2, wherein
the substrate and the protruding portion are integrally formed.

5. The cell culture apparatus according to claim 1, wherein
the cells include microorganisms or animal cells.

6. The cell culture apparatus according to claim 1, further comprising:
a main body where the dispersion fluid is accommodated, the main body holding the substrate and the mesh member; and
an introduction flow channel for introduction of the specific solution, the introduction flow channel being fluidly connected to the main body.

7. The cell culture apparatus according to claim 1, further comprising a lid arranged above the mesh member in the vertical direction, wherein
in the lid, an opening smaller in width than the mesh member is formed.

8. The cell culture apparatus according to claim 7, further comprising a translation mechanism that changes a position of the mesh member with respect to the opening of the lid in a horizontal direction.

9. A culture unit comprising:
the cell culture apparatus according to claim 1;
a microscope for observation of the droplets in the cell culture apparatus; and
a collection apparatus that individually collects the droplets.

10. A cell culture method comprising:
generating a dispersion fluid in which droplets in which a culture fluid containing cells is sealed are dispersed in a specific solution, the specific solution being larger in specific gravity than the culture fluid;
holding, at a substrate including a main surface, the dispersion fluid on the main surface; and
arranging a mesh member away from the main surface of the substrate, wherein
in the mesh member, a plurality of through holes are formed where the droplets are to be trapped while the mesh member is located above the substrate in a vertical direction, the droplets having been introduced to the substrate and having risen.

11. The cell culture method according to claim 10, wherein
the arranging the mesh member includes arranging the mesh member above a protruding portion that is arranged on the substrate and protrudes upward from the substrate.

12. The cell culture method according to claim 10, further comprising:
observing the droplets trapped in the mesh member; and
individually collecting the droplets.
